# EUROPÄISCHE PATENTSCHRIFT

(11) **EP 3 623 518 B1**
(45) Veröffentlichungstag und Bekanntmachung des Hinweises auf die Patenterteilung: **09.11.2022**
(21) Anmeldenummer: 19194009.7
(22) Anmeldetag: 28.08.2019
(51) Int. Cl.: D06F 33/46, A61L 2/07, D06F 33/72, A47L 15/00, D06F 105/30, D06F 58/38, D06F 105/28, D06F 103/02, D06F 103/12, D06F 105/02, D06F 25/00

(54) **VERFAHREN ZUM ANSTEUERN EINES ERWÄRMENS EINES REINIGUNGSGUTS WÄHREND EINES REINIGUNGSVORGANGS IN EINEM REINIGUNGSGERÄT**
METHOD FOR CONTROLLING THE HEATING OF A CLEANING MATERIAL DURING A CLEANING PROCESS IN A CLEANING DEVICE
PROCÉDÉ PERMETTANT DE COMMANDER LE CHAUFFAGE D'UN PRODUIT À NETTOYER AU COURS D'UN PROCESSUS DE NETTOYAGE DANS UN APPAREIL DE NETTOYAGE

(30) Priorität: 14.09.2018 DE 102018122461
(43) Veröffentlichungstag der Anmeldung: 18.03.2020
(73) Patentinhaber: Miele & Cie. KG, 33332 Gütersloh (DE)
(72) Erfinder: Bicker, Rainer, 33415 Verl (DE); Drücker, Markus, 33335 Gütersloh (DE); Kortenjann, Magnus, 48291 Telgte (DE); Templin, Ralf, 33647 Bielefeld (DE)

(56) Entgegenhaltungen:
- EP-A1- 2 503 048
- EP-A1- 3 375 344
- DE-A1-102013 210 129
- DE-A1-102017 103 498
- US-A1- 2009 255 299
- US-A1- 2012 180 534

## Beschreibung

Die Erfindung betrifft ein Verfahren und ein Steuergerät zum Ansteuern eines Erwärmens eines Reinigungsguts während eines Reinigungsvorgangs in einem Reinigungsgerät und ein Reinigungsgerät.

Es ist möglich, in einem Reinigungsgerät wie einer Waschmaschine aufgenommenes Reinigungsgut zeitgesteuert aufzuheizen, in der Waschmaschine beispielsweise um Knitter in der Wäsche zu reduzieren. Dieses Aufheizen kann energieintensiv sein, da es unabhängig von einer Beladung des Reinigungsgeräts durchgeführt werden kann.

Aus der EP 2 503048 A1 ist ein Verfahren bekannt, bei dem nach dem Hauptreinigungsvorgang die Wäsche erwärmt wird, um beim Schleudern mehr Wasser aus dem Stoff zu treiben. Die Erwärmung erfolgt hierbei in Abhängigkeit der Wäscheart oder der Beladungsmenge. Auch aus der US 2012/180534 A1, der DE 10 2013 210129 A1, der DE 10 2017 103498 A1 und der EP 3 375344 A1 sind solche Verfahren bekannt.

Die US 2009/0255299 A1 offenbart ein Verfahren zum Reinigen von Wäsche, welches eine nachträgliche Ozonbehandlung umfasst. Hierbei wird die Intensität der Ozonzugabe in Abhängigkeit der Wäsche geregelt, um das Austreten von ungesättigtem, also noch reaktiven Ozongas zu vermeiden.

Der Erfindung stellt sich die Aufgabe ein verbessertes Verfahren und ein verbessertes Steuergerät zum Ansteuern eines Erwärmens eines Reinigungsguts während eines Reinigungsvorgangs in einem Reinigungsgerät und ein verbessertes Reinigungsgerät zu schaffen.

Erfindungsgemäß wird diese Aufgabe durch ein Verfahren und ein Steuergerät zum Ansteuern eines Erwärmens eines Reinigungsguts während eines Reinigungsvorgangs in einem Reinigungsgerät und durch ein Reinigungsgerät mit den Schritten bzw. Merkmalen der Hauptansprüche gelöst. Vorteilhafte Ausgestaltungen und Weiterbildungen der Erfindung ergeben sich aus den nachfolgenden Unteransprüchen.

Die mit der Erfindung erreichbaren Vorteile bestehen darin, dass ein in dem Reinigungsgerät aufgenommenes Reinigungsgut nach einem Hauptreinigungsvorgang erwärmt werden kann, wodurch eine Feuchte des Reinigungsguts reduziert werden kann. Dies ist vorteilhafterweise energiesparend im Hinblick auf einen nachfolgenden Trockenvorgang. Durch das Erwärmen des Reinigungsgut nach dem Hauptreinigungsvorgang kann zudem eine Keimabtötung durch eine erhöhte Wassertemperatur erfolgen, was vorteilhafterweise die Hygiene des Reinigungsguts erhöhen kann. Außerdem kann das Erwärmen des Reinigungsguts vorteilhafterweise einen Entnahmekomfort eines Anwenders beim Entnehmen des Reinigungsgut aus dem Reinigungsgerät erhöhen.

Auch wenn der beschriebene Ansatz anhand eines Haushaltgerät beschrieben wird, kann der hier beschriebene Ansatz entsprechend im Zusammenhang mit einem gewerblichen oder professionellen Gerät, beispielsweise einem medizinischen Gerät, wie einem Reinigungsoder Desinfektionsgerät, einem Kleinsterilisator, einem Großraumdesinfektor oder einer Container-Waschanlage eingesetzt werden.

Ein Verfahren zum Ansteuern eines Erwärmens eines Reinigungsguts während eines Reinigungsvorgangs in einem Reinigungsgerät weist zumindest einen Schritt des Einlesens und einen Schritt des Bestimmens auf. Im Schritt des Einlesens wird ein Beladungssignal eingelesen, das zumindest einen Beladungsparameter von in dem Reinigungsgerät aufgenommenem Reinigungsgut repräsentiert. Im Schritt des Bestimmens wird unter Verwendung des Beladungssignals ein Erwärmungssignal bestimmt, um ein Erwärmen des in dem Reinigungsgerät aufgenommenen Reinigungsguts nach einem letztmaligen Reinigungsvorgang mit einem Pflegemittel anzusteuern.

Bei dem Reinigungsgerät kann es sich beispielsweise um eine Waschmaschine oder um einen Waschtrockner handeln, oder um ein Großraum-Reinigungs- und Desinfektionsgerät wie eine Container- oder Rollwagenwaschanlage. Das Reinigungsgut kann beispielsweise Wäsche sein. Das Reinigungsgut kann während eines Reinigungsvorgangs erwärmt werden, beispielsweise während eines Spülvorgangs, der anschließend an einen Hauptwaschgang durchgeführt werden kann, also nach einem letztmaligen Reinigungsvorgang mit einem Pflegemittel wie einem chemischen Waschmittel. Das Erwärmen des Reinigungsguts kann beispielsweise auch nach dem Spülvorgang vor, während oder nach einem Endschleudervorgang erfolgen. Das Beladungssignal kann ein elektrisches Signal oder ein Funksignal sein, das beispielsweise von einer Sensoreinrichtung des Reinigungsgeräts bereitgestellt werden kann. Unter dem Beladungsparameter kann beispielsweise eine Beladungsmenge verstanden werden, oder eine Art des in dem Reinigungsgerät aufgenommenen Reinigungsguts, beispielsweise eine Textilienart, oder ein bestimmtes Reinigungsverhalten des Reinigungsguts, wie beispielsweise eine Saugfähigkeit. Das Erwärmungssignal kann an eine Schnittstelle zu einem Heizelement oder zu einer Sensoreinrichtung des Reinigungsgeräts, beispielsweise einem Temperatursensor, bereitgestellt werden. Dazu kann das Erwärmungssignal beispielsweise ausgebildet sein, ein Heizelement anzusteuern, beispielsweise einen Strahlungsheizkörper oder ein Heißluftgebläse oder eine Dampferzeugungseinrichtung, um Heizenergie in das Reinigungsgut zu bringen. Zum Ansteuern des Erwärmens des Reinigungsguts kann beispielsweise ein Aufnahmebereich des Reinigungsguts wie eine Wäschetrommel beheizt werden, beispielswiese mittels Dampf- oder Trockenheizen. Das Erwärmungssignal kann ausgebildet sein, das Heizen anzusteuern. Zudem kann das Erwärmungssignal ausgebildet sein, ein Einstellen einer bestimmten Zieltemperatur des Heizens anzusteuern, beispielsweise eine Zieltemperatur im Bereich zwischen 30 und 95 Grad Celsius. Vorteilhafterweise kann das Erwärmen des Reinigungsgutes entsprechend unter Verwendung einer bereits vorhandenen Heizvorrichtung des Reinigungsgeräts ausgeführt werden, was kostensparend ist und eine kompakte Bauweise ermöglicht.

Gemäß einer Ausführungsform kann das Verfahren auch einen Schritt des Ermittelns des Beladungssignals aufweisen. Als Beladungsparameter kann eine Beladungsmenge und zusätzlich oder alternativ eine Art des in dem Reinigungsgerät aufgenommenen Reinigungsguts ermittelt werden. Insbesondere kann auch unter Verwendung einer vorbestimmten Verarbeitungsvorschrift eine in dem Reinigungsgut befindliche oder aufgenommene Wassermenge ermittelt werden. Die Beladungsmenge kann beispielsweise mittels eine Masseträgheitsverfahrens oder mittels eines Wiegeverfahrens ermittelt werden. Die Art des Reinigungsguts kann beispielsweise mittels eines Saugverhaltens des Reinigungsguts unter Verwendung eines Drucksensors ermittelt werden. Vorteilhafterweise kann das Erwärmen des Reinigungsguts durch ein Berücksichtigen der Beladungsmenge energiesparend durchgeführt werden. Zudem kann durch ein Ermitteln der Art des Reinigungsguts eine Temperaturempfindlichkeit des Materials des Reinigungsguts ermittelt werden vorteilhaft ein materialschonendes Erwärmen des Reinigungsguts ermöglicht.

Das Verfahren kann gemäß einer Ausführungsform auch einen Schritt des Ausgebens eines Belüftungssignals an eine Schnittstelle zu einer Belüftungseinrichtung des Reinigungsgeräts umfassen. Das Belüftungssignal kann unter Verwendung des Erwärmungssignals bestimmt werden und dazu ausgebildet sein, die Belüftungseinrichtung anzusteuern. Die Belüftungseinrichtung kann beispielsweise einen Ventilator umfassen und dazu ausgebildet sein, Luft durch den Aufnahmebereich des Reinigungsguts, beispielsweise die Wäschetrommel, zu transportieren. Das Belüftungssignal kann ausgebildet sein, die Belüftungseinrichtung zu aktivieren oder zu deaktivieren, oder eine Gebläseleistung einzustellen. Die Betriebsdauer der Belüftungseinrichtung kann in Abhängigkeit des Beladungsparameters und zusätzlich oder alternativ einer Restfeuchte des Reinigungsguts und zusätzlich oder alternativ einer Temperatur des Reinigungsguts oder einer Umgebungstemperatur des Reinigungsgutes eingestellt werden. Vorteilhafterweise ist es durch einen Einsatz der Belüftungseinrichtung beispielsweise in Verbindung mit einer Waschmaschine möglich, eine durch das Endschleudern einer Wäsche erreichbare oder erreichte Restfeuchte weiter zu reduzieren, was energieeffizient ist.

Zudem weist das Verfahren erfindungsgemäß einen Schritt des Generierens eines Kaltwassersignals auf. Das Kaltwassersignal kann unter Verwendung des Erwärmungssignals generiert werden und dazu ausgebildet sein, ein Einleiten von kaltem Wasser in einen Sumpf eines Pflegemittelbehälters des Reinigungsgeräts anzusteuern. Mittels des Kaltwassersignals kann beispielsweise nach dem Erwärmen des Reinigungsguts Wasser in den Sumpf eingeleitet werden. Die im Heizschritt mit aufgewärmte nicht gesättigte Luft kann Wasser von dem Reinigungsgut aufnehmen. Bei einer Waschmaschine kann durch eine Reversierbewegung der Waschtrommel Luft bis zur kalten Wasseroberfläche transportiert werden. Dort kann die Luft unter den Taupunkt abkühlen, so dass der in der Luft gasförmig vorliegende Wasserdampf kondensiert. Die entfeuchtete Luft erwärmt sich an der Wäsche und den warmen Bauteilen des Gerätes wieder und kann Wäschefeuchte aufnehmen. Das führt vorteilhafterweise zu einer Verringerung der Wäschefeuchte, was beispielsweise bei einem Trocknen des Reinigungsguts energiesparend ist.

Im Schritt des Einlesens kann gemäß einer Ausführungsform ein Temperatursignal eingelesen werden, das eine erfasste Temperatur des in dem Reinigungsgerät aufgenommenen Reinigungsguts repräsentiert. In diesem Fall kann im Schritt des Bestimmens das Erwärmungssignal unter Verwendung des Temperatursignals bestimmt werden. Das Temperatursignal kann beispielsweise von einem Temperatursensor des Reinigungsgeräts bereitgestellt werden. Das Temperatursignal kann zusätzlich oder alternativ eine erfasste Umgebungstemperatur des in dem Reinigungsgerät aufgenommenen Reinigungsguts repräsentieren. Das Erwärmungssignal kann derart ausgebildet sein, ein Erwärmen des Reinigungsguts um die Differenz zwischen der erfassten Temperatur und der Zieltemperatur anzusteuern. Vorteilhafterweise kann das Erwärmen des Reinigungsguts auf diese Weise besonders energieeffizient erfolgen.

Ferner kann im Schritt des Bestimmens gemäß einer Ausführungsform eine Heizenergiemenge zum Bestimmen des Erwärmungssignals bestimmt werden. Die Heizenergiemenge kann unter Verwendung des Beladungssignals, des Temperatursignals und einer Zuordnungsvorschrift bestimmt werden. Die Zuordnungsvorschrift kann eine Heizenergiemenge unter Berücksichtigung einer Temperatur des Reinigungsguts und einer Restwassermenge in dem Reinigungsgut abbilden. Vorteilhafterweise ist es somit möglich die Energiemenge zu ermitteln, die zum Erreichen der Zieltemperatur des Reinigungsgutes erforderlich ist.

Der hier vorgestellte Ansatz schafft ferner ein Steuergerät, das ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form eines Steuergeräts kann die der Erfindung zugrunde liegende Aufgabe schnell und effizient gelöst werden.

Das Steuergerät kann ausgebildet sein, um Eingangssignale einzulesen und unter Verwendung der Eingangssignale Ausgangssignale zu bestimmen und bereitzustellen. Ein Eingangssignal kann beispielsweise ein über eine Eingangsschnittstelle des Steuergeräts einlesbares Sensorsignal darstellen. Ein Ausgangssignal kann ein Steuersignal oder ein Datensignal darstellen, das an einer Ausgangsschnittstelle des Steuergeräts bereitgestellt werden kann. Das Steuergerät kann ausgebildet sein, um die Ausgangssignale unter Verwendung einer in Hardware oder Software umgesetzten Verarbeitungsvorschrift zu bestimmen. Beispielsweise kann das Steuergerät dazu eine Logikschaltung, einen integrierten Schaltkreis oder ein Softwaremodul umfassen und beispielsweise als ein diskretes Bauelement realisiert sein oder von einem diskreten Bauelement umfasst sein.

Mit diesem Ansatz wird zudem ein Reinigungsgerät mit einer Ausführungsform des vorstehend genannten Steuergeräts vorgestellt.

Von Vorteil ist auch ein Computer-Programmprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann. Wird das Programmprodukt oder Programm auf einem Computer oder einem Steuergerät ausgeführt, so kann das Programmprodukt oder Programm zur Durchführung, Umsetzung und/oder Ansteuerung der Schritte des Verfahrens nach einer der vorstehend beschriebenen Ausführungsformen verwendet werden.

Ein Ausführungsbeispiel der Erfindung ist in den Zeichnungen rein schematisch dargestellt und wird nachfolgend näher beschrieben. Es zeigt
- Figur 1: eine schematische Darstellung eines Steuergeräts zum Ansteuern eines Erwärmens eines Reinigungsguts während eines Reinigungsvorgangs in einem Reinigungsgerät gemäß einem Ausführungsbeispiel;
- Figur 2: eine schematische Darstellung eines Steuergeräts zum Ansteuern eines Erwärmens eines Reinigungsguts während eines Reinigungsvorgangs in einem Reinigungsgerät gemäß einem Ausführungsbeispiel;
- Figur 3: eine schematische Darstellung eines Reinigungsgeräts mit einem Steuergerät zum Ansteuern eines Erwärmens eines Reinigungsguts während eines Reinigungsvorgangs in dem Reinigungsgerät gemäß einem Ausführungsbeispiel; und
- Figur 4: ein Ablaufdiagramm eines Verfahrens zum Ansteuern eines Erwärmens eines Reinigungsguts während eines Reinigungsvorgangs in einem Reinigungsgerät gemäß einem Ausführungsbeispiel.

In der nachfolgenden Beschreibung günstiger Ausführungsbeispiele des vorliegenden Ansatzes werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

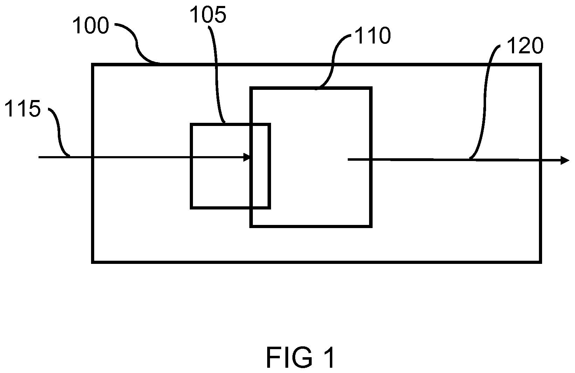

Figur 1 zeigt eine schematische Darstellung eines Steuergeräts 100 zum Ansteuern eines Erwärmens eines Reinigungsguts während eines Reinigungsvorgangs in einem Reinigungsgerät gemäß einem Ausführungsbeispiel. Das Steuergerät 100 umfasst hier eine Einleseeinrichtung 105 und eine Bestimmungseinrichtung 110. Die Einleseeinrichtung 105 ist ausgebildet, ein Beladungssignal 115 einzulesen. Das Beladungssignal 115 repräsentiert zumindest einen Beladungsparameter von in dem Reinigungsgerät aufgenommenem Reinigungsgut. Die Bestimmungseinrichtung 110 ist ausgebildet unter Verwendung des Beladungssignals 115 ein Erwärmungssignal 120 zu bestimmen, um ein Erwärmen des in dem Reinigungsgerät aufgenommenen Reinigungsguts nach einem letztmaligen Reinigungsvorgang mit einem Pflegemittel anzusteuern.

Das Erwärmen des Reinigungsguts erfolgt nach einem letztmaligen Reinigungsvorgang mit einem Pflegemittel, beispielsweise im Anschluss an einen Hauptreinigungsvorgang, durch ein Ansteuern eines Heizelements mittels des Erwärmungssignals 120. Bei einer Waschmaschine kann das bedeuten, dass das Erwärmen während eines Spülens einer Wäsche nach einem Hauptwaschgang erfolgt. Das für den Spülprozess einlaufende Wasser kann erhitzt werden, sodass sich die Wäschetemperatur nicht mit jedem Wassereinlauf immer mehr der kalten Wassereinlauftemperatur annähert, und somit bei Programmende sehr niedrig ist. Vorteilhafterweise führt das Erwärmen des Reinigungsguts zu einem angenehmen Gefühl beim Entladen der Wäsche aus der Waschmaschine.

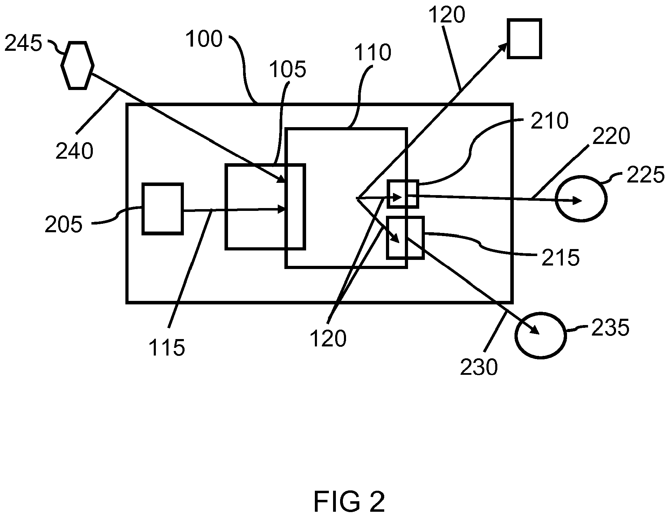

Figur 2 zeigt eine schematische Darstellung eines Steuergeräts 100 zum Ansteuern eines Erwärmens eines Reinigungsguts während eines Reinigungsvorgangs in einem Reinigungsgerät gemäß einem Ausführungsbeispiel. Das Steuergerät 100 umfasst hier außer der Einleseeinrichtung 105 und der Bestimmungseinrichtung 110 eine Ermittlungseinrichtung 205, eine Ausgabeeinrichtung 210 und eine Generierungseinrichtung 215.

Die Ermittlungseinrichtung 205 ist gemäß dem hier gezeigten Ausführungsbeispiel dazu ausgebildet, das Beladungssignal 115 zu ermitteln. Als Beladungsparameter des Beladungssignals 115 werden eine Beladungsmenge und zusätzlich oder alternativ ein Art des in dem Reinigungsgerät aufgenommenen Reinigungsguts ermittelt. Das Beladungssignal 115 wird insbesondere ermittelt, um unter Verwendung einer vorbestimmten Verarbeitungsvorschrift eine in dem Reinigungsgut befindliche Wassermenge zu ermitteln. Die Ermittlungseinrichtung 205 ist zudem ausgebildet, das Beladungssignal 115 bereitzustellen. Die Ermittlungseinrichtung 205 ist optional ausgebildet, die Beladungsmenge mittels eines Massenträgheitsverfahrens oder eines Wiegeverfahrens zu bestimmen. Zusätzlich oder alternativ ist die Ermittlungseinrichtung 205 optional dazu ausgebildet, die Beladungsart anhand eines Saugverhaltens des Reinigungsguts zu bestimmen.

Gemäß dem hier gezeigten Ausführungsbeispiel ist die Ausgabeeinrichtung 210 dazu ausgebildet, ein Belüftungssignal 220 an eine Schnittstelle 225 zu einer Belüftungseinrichtung des Reinigungsgeräts auszugeben. Das Belüftungssignal 220 wird unter Verwendung des Erwärmungssignals 120 bestimmt und ist dazu ausgebildet, die Belüftungseinrichtung anzusteuern.

Dies ist beispielsweise bei einer Verwendung des Steuergeräts 100 in Verbindung mit einer Waschmaschine als Reinigungsgerät vorteilhaft: Die Wäsche kann im Endschleuderprozess lediglich auf einen physikalisch minimal möglichen Restfeuchtewert, die Grenzrestfeuchte, entfeuchtet werden. Die Trommeldrehzahl der Wäschetrommel ist technisch begrenzt, da bei einer weiteren Erhöhung der Trommeldrehzahl negative Effekte wie Knitterbildung zunehmen. Durch das Ansteuern der Belüftungseinrichtung mittels des Belüftungssignals 220 ist es möglich, den Wert der Restfeuchte weiter zu reduzieren. Das Steuergerät 100 ist ausgebildet, mittels des Erwärmungssignals 120 die Energiemenge beladungsmengenabhängig zu regeln, die am Ende eines Waschprogrammes zum Erwärmen der Wäsche eingesetzt wird. Optional wird mittels des Belüftungssignals 220 zudem die Restfeuchte der Wäsche reduziert.

Die Generierungseinrichtung 215 ist gemäß dem hier gezeigten Ausführungsbeispiel dazu ausgebildet, unter Verwendung des Erwärmungssignals 120 ein Kaltwassersignal 230 zu generieren. Das Kaltwassersignal 230 ist dazu ausgebildet, ein Einleiten von kaltem Wasser in einen Sumpf eines Pflegemittelbehälters des Reinigungsgeräts anzusteuern. Hier wird das Kaltwassersignal 230 beispielhaft an eine Schnittstelle 235 zu einem Wasserzulauf des Reinigungsgeräts bereitgestellt. Das Kaltwassersignal 230 ist optional ausgebildet, das Einleiten des kalten Wassers nach dem Erwärmen des Reinigungsgutes anzusteuern. Die im Heizschritt mit aufgewärmte nicht gesättigte Luft kann Wasser von dem Reinigungsgut aufnehmen. Durch ein Transportieren der Luft zu dem kalten Wasser in dem Sumpf kann die Luft abkühlt werden, sodass der in der Luft gasförmig vorliegende Wasserdampf kondensiert. Die Luft kann nun erneut Feuchtigkeit aufnehmen, was eine Restfeuchte des Reinigungsguts reduziert.

Die Einleseeinrichtung 105 ist gemäß dem hier gezeigten Ausführungsbeispiel zudem dazu ausgebildet, ein Temperatursignal 240 einzulesen. Das Temperatursignal 240 repräsentiert eine erfasste Temperatur des in dem Reinigungsgerät aufgenommenen Reinigungsguts. In diesem Fall ist die Bestimmungseinrichtung 110 dazu ausgebildet, das Erwärmungssignal 120 unter Verwendung des Temperatursignals 240 zu bestimmen. Hier wird das Temperatursignal 240 beispielhaft von einem Temperatursensor 245 bereitgestellt.

Zudem ist die Bestimmungseinrichtung 110 gemäß dem hier gezeigten Ausführungsbeispiel dazu ausgebildet, unter Verwendung des Beladungssignals 115, des Temperatursignals 240 und einer Zuordnungsvorschrift eine Heizenergiemenge zum Bestimmen des Erwärmungssignals 120 zu bestimmen. Die Zuordnungsvorschrift bildet eine Heizenergiemenge unter Berücksichtigung einer Temperatur des Reinigungsguts und einer Restwassermenge in dem Reinigungsgut ab.

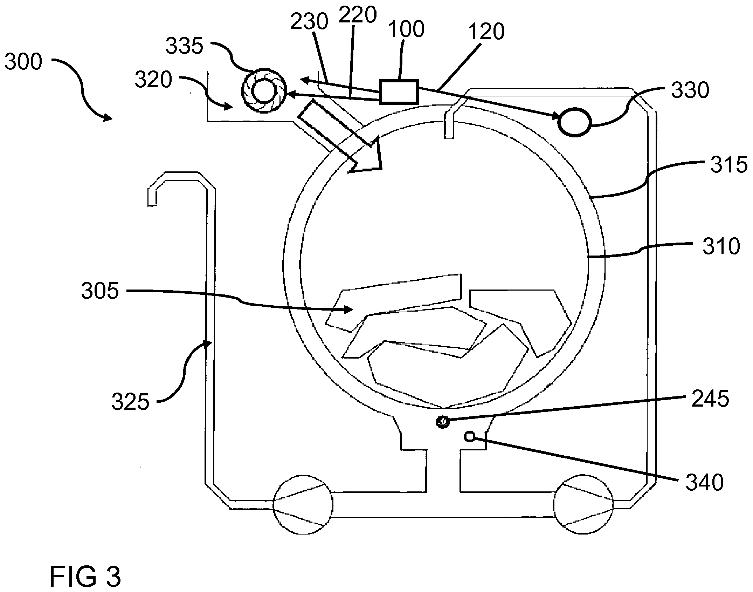

Figur 3 zeigt eine schematische Darstellung eines Reinigungsgeräts 300 mit einem Steuergerät 100 zum Ansteuern eines Erwärmens eines Reinigungsguts 305 während eines Reinigungsvorgangs in dem Reinigungsgerät 300 gemäß einem Ausführungsbeispiel. Das hier gezeigte Steuergerät 100 ähnelt oder entspricht dem anhand der vorhergehenden Figuren beschriebenen Steuergerät. Als Reinigungsgerät 300 ist hier beispielhaft eine Waschmaschine gezeigt. In einer Trommel 310 der Waschmaschine sind als Reinigungsgut 305 Wäschestücke aufgenommen. Um die Trommel 310 umlaufend ist ein Laugenbehälter 315 angeordnet. Der Laugenbehälter 315 kann auch als Pflegemittelbehälter 315 bezeichnet werden. Das Reinigungsgerät 300 weist zudem einen Wasserzulauf 320 und einen Ablaufschlauch 325 auf. Zudem umfasst das Reinigungsgerät 300 ein Heizelement 330 und eine Belüftungseinrichtung 335, ein Gebläse. Unterhalb der Trommel 310 sind beispielhaft der Temperatursensor 245 und ein Drucksensor 340 angeordnet. Das Steuergerät 100 ist gemäß dem hier gezeigten Ausführungsbeispiel ausgebildet, mittels des Erwärmungssignals 120 das Heizelement 330 anzusteuern. Zudem ist das Steuergerät 100 ausgebildet, mittels des Belüftungssignals 220 die Belüftungseinrichtung 335 anzusteuern und mittels des Kaltwassersignals 230 den Wasserzulauf 320 anzusteuern.

Im Folgenden wird beispielhaft eine Verwendung eines Ausführungsbeispiels des Steuergeräts 100 in Verbindung mit der Waschmaschine als Reinigungsgerät 300 erläutert: Das Steuergerät 100 ist ausgebildet, die Beladungsmenge und die Beladungsart des Reinigungsgeräts 300 zu ermitteln. Die Beladungsmenge wird beispielsweise mittels eines Wiegeverfahrens oder eines Massenträgheitsverfahrens bestimmt. Die Beladungsart wird anhand eines Saugverhaltens des Reinigungsguts 305 bestimmt. Saugfähige Wäsche saugt Wasser schneller auf als wenig saugfähige Wäsche. Mittels eines analogen Drucksensors 340 ist ein Absinken eines Wasserstandes im Laugenbehälter 315 bestimmbar. Ebenso ist durch eine Zeitmessung die Absinkgeschwindigkeit des Wasserstandes im Laugenbehälter 315 ermittelbar, wodurch auf das Saugverhalten und die Wäscheart geschlossen werden kann. Alternativ ist die Wäscheart auch durch das Massenträgheitsverfahren bestimmbar. So werden unterschiedliche Wäschearten verschieden stark verpresst, wenn sie beschleunigt werden. Die unterschiedliche Verpressung erzeugt Unterschiede im Massenträgheitsmoment, wodurch auf die Wäscheart geschlossen werden kann. Sich stark verpressende Wäsche ist saugfähiger als weniger stark verpressende Wäsche. Sind Wäschemenge und Wäscheart bekannt, so kann mittels eines empirischen mathematischen Algorithmus während des Endschleudervorganges die in der Wäsche verbleibende Wassermenge abgeschätzt werden.

Der in dem Reinigungsgerät 300, im Waschautomaten, befindliche Temperatursensor 245 misst nach dem Endschleudern und bevor die Heizphase beginnt die Wäschetemperatur. Das Steuergerät 100 ist gemäß einem Ausführungsbeispiel dazu ausgebildet, basierend auf der in der Wäsche nach dem Endschleudern verbliebenen Wassermenge und der Wassertemperatur diejenige Heizenergiemenge zu ermitteln, die erforderlich ist, um eine gewünschte Temperatur in der Wäsche zu erreichen. Die Bestimmung der Energiemenge kann durch einen weiteren empirischen mathematischen Algorithmus durchgeführt werden, in dem die Eingangsgrößen Restwassermenge in der Wäsche und Temperatur zu einer Heizenergiemenge verrechnet werden. Der Heizprozess ist alternativ beispielweise auch durch den Temperatursensor 245 steuerbar. Ist eine gewünschte Temperatur, die durch den Temperatursensor 245 erfasst wird, erreicht, wird das Heizelement 330 abgeschaltet. Da die Wassermenge in der Wäsche zur diesem Zeitpunkt sehr niedrig ist, ist auch nur sehr wenig Heizenergie notwendig, um die gewünschte Temperatur zu erreichen. Dies ist energetisch sehr effizient. Gemäß dem hier gezeigten Ausführungsbeispiel wird das Heizelement 330 mittels des Erwärmungssignals 120 angesteuert, das von dem Steuergerät bereitgestellt wird. Die gewünschte Temperatur kann beispielsweise auf 60 Grad Celsius festgelegt sein, um eine gute keimabtötende Wirkung zu erzielen. Eine noch bessere Wirkung wird bei einer Wäschetemperatur von 95°C erreicht. Das Ergebnis der Bestimmung der Wäscheart (empfindliche Wäsche = wenig saugfähig, unempfindliche Wäsche = stark saugfähig) kann beim Bestimmen des Erwärmungssignals 120 berücksichtigt werden, um anzusteuern, welche Temperatur eingestellt wird. Eine ausreichende Reversierdauer der Trommel 310 bei dieser Temperatur stellt sicher, dass die Temperatur gleichmäßig in der Wäsche vorhanden ist und die Wärme gleichmäßig verteilt wird. Die Temperatur kann auch auf niedrigere Werte, z.B. 30 Grad Celsius, festgelegt werden, um eine angenehme Entnahmetemperatur zu erzeugen. Das Erwärmen des Reinigungsguts 305 ist mittels verschiedener Heizverfahren realisierbar: Dampf zum Heizen (Komfortglätten, mit einer Dampfeinheit auf einem Trommelmantel der Trommel 310 von außen), Dampferzeugung durch Anspritzen einer heißen Platte, Trockenheizen, Induktion, Heizen mittels eines Heißluftgebläses, ein Strahlungsheizkörper oder eine Wärmepumpe Luft-Luft.

Ein Reduzieren der nach dem Endschleudern erreichten Restfeuchte in der Wäsche ist durch ein Ansteuern der Belüftungseinrichtung 335 mittels des Belüftungssignals 220 möglich. Nachdem die gewünschte Temperatur durch den Heizvorgang erreicht wurde, wird die Belüftungseinrichtung 335, das Gebläse, eingeschaltet, so dass ein Lufttransport durch die Trommel 310 erfolgt. Die Belüftungseinrichtung 335 ist hier beispielhaft im Bereich des Wasserzulaufs 320 angeordnet. Ein Reversieren der Waschtrommel 310 während des Betreibens der Belüftungseinrichtung 335 ist dabei vorteilhaft: Damit wird die Kontaktfläche zwischen trockener Raumluft, die sich in der Trommel 310 erwärmt, und warmer Wäsche intensiviert. Die Betriebsdauer des Betreibens der Belüftungseinrichtung 335 kann in Abhängigkeit der Wäschemenge, der Wäscheart, der Raumtemperatur, der Wäschetemperatur, der zu Start vorhandenen Restfeuchte und der zu erreichenden Wäschefeuchte mittels des Steuergeräts 100 des Reinigungsgeräts 300, hier des Waschautomaten, festgelegt werden. Auf diese Weise sind Restfeuchten erreichbar, die durch ein mechanisches Trennverfahren wie das Endschleudern nicht erreicht werden können. Dazu ist die Belüftungseinrichtung 335 beispielsweise im Einspülkasten, in dem Wasserzulauf 320, angeordnet und bläst über die Waschmittelschublade von außen angesaugte Luft über den Wasserzulauf 320 in den Laugenbehälter 315. Die Luft wird durch Undichtigkeiten, wie z.B. den Ablaufschlauch 325 oder Öffnungen im Türdichtring zum Geräteinnenraum des Reinigungsgeräts 300 ungerichtet wieder aus dem Laugenbehälter 315 transportiert.

Zusätzlich oder alternativ ist das Steuergerät 100 ausgebildet, nach dem Heizen mittels des Kaltwassersignals 230 ein Einleiten von kaltem Wasser in den Sumpf des Laugenbehälters 315 anzusteuern. Die bei Heizen mit aufgewärmte nicht gesättigte Luft in der Trommel 310 kann Wasser von der Wäsche aufnehmen. Durch eine Reversierbewegung der Waschtrommel 310 wird diese Luft bis zur kalten Wasseroberfläche transportiert. Dort kühlt sie unter den Taupunkt ab, so dass der in der Luft gasförmig vorliegende Wasserdampf kondensiert. Die entfeuchtete Luft erwärmt sich an der Wäsche und den warmen Bauteilen des Reinigungsgeräts 300 wieder und kann Wäschefeuchte aufnehmen. Das führt zu einer Verringerung der Wäschefeuchte. Darüber hinaus ist es vorteilhaft, dass sich das Sumpfwasser in dem Laugenbehälter 315 durch die bei der Kondensation freiwerdende Energie erwärmt. Dieses erwärmte Sumpfwasser kann für den folgenden Waschprozess verwendet werden, so dass sich die für das Erwärmen notwendige Heizenergie im folgenden Waschprozess reduziert. Dies spart Energie.

Durch das Verwenden des Steuergeräts 100 ist es vorteilhafterweise möglich, durch eine Keimabtötung bei erhöhter Wäschetemperatur bei gleichzeitig energieeffizientem Einsatz von Heizenergie eine erhöhte Hygiene zu erreichen. Zudem ergibt sich für einen Benutzer des Reinigungsgeräts 300 einen verbesserten Entnahmekomfort durch eine angenehme Wäschetemperatur, indem die Wäschetemperatur in einen für den Nutzer deutlich angenehmeren Temperaturbereich verschoben wird. und eine Lockerheit der Wäsche, die durch das Drehen der Trommel 310 nach der Erwärmungsphase erreicht wird. Vorteilhaft ist auch die mittels des Ansteuerns der Belüftungseinrichtung 335 erreichbare reduzierte Wäschefeuchte, die eine Energieersparnis im sich anschließenden Trockenprozess zur Folge hat.

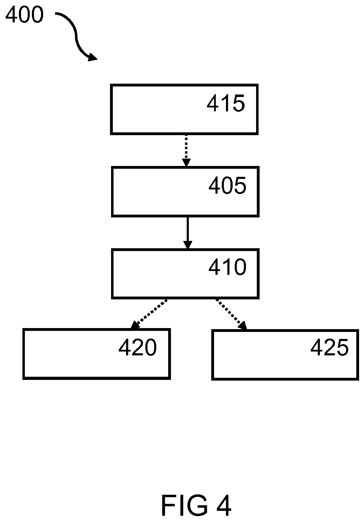

Figur 4 zeigt ein Ablaufdiagramm eines Verfahrens 400 zum Ansteuern eines Erwärmens eines Reinigungsguts während eines Reinigungsvorgangs in einem Reinigungsgerät gemäß einem Ausführungsbeispiel. Das Verfahren 400 weist zumindest einen Schritt 405 des Einlesens und einen Schritt 410 des Bestimmens auf. Im Schritt 405 des Einlesens wird ein Beladungssignal eingelesen, das zumindest einen Beladungsparameter von in dem Reinigungsgerät aufgenommenem Reinigungsgut repräsentiert. Im Schritt 410 des Bestimmens wird unter Verwendung des Beladungssignals ein Erwärmungssignal bestimmt, um ein Erwärmen des in dem Reinigungsgerät aufgenommenen Reinigungsguts nach einem letztmaligen Reinigungsvorgang mit einem Pflegemittel anzusteuern.

Gemäß einem Ausführungsbeispiel umfasst das Verfahren 400 einen Schritt 415 des Ermittelns, der vor dem Schritt 405 des Einlesens durchgeführt wird. Im Schritt 415 des Ermittelns wird das Beladungssignal ermittelt. Als Beladungsparameter werden eine Beladungsmenge und zusätzlich oder alternativ eine Art des in dem Reinigungsgerät aufgenommenen Reinigungsguts ermittelt. Die Beladungsparameter werden insbesondere ermittelt, um unter Verwendung einer vorbestimmten Verarbeitungsvorschrift eine in dem Reinigungsgut befindliche Wassermenge zu ermitteln.

Zudem umfasst das Verfahren 400 gemäß einem Ausführungsbeispiel einen Schritt 420 des Ausgebens. Im Schritt 420 des Ausgebens wird ein Belüftungssignal an eine Schnittstelle zu einer Belüftungseinrichtung des Reinigungsgeräts ausgegeben. Das Belüftungssignal wird unter Verwendung des Erwärmungssignals bestimmt. Das Belüftungssignal ist ausgebildet, die Belüftungseinrichtung anzusteuern. Der Schritt 420 des Ausgebens wird optional nach dem Schritt 410 des Bestimmens ausgeführt.

Außerdem umfasst das Verfahren 400 gemäß einem Ausführungsbeispiel einen Schritt 425 des Generierens. Im Schritt 425 des Generierens wird unter Verwendung des Erwärmungssignals ein Kaltwassersignal generiert. Das Kaltwassersignal ist ausgebildet, ein Einleiten von kaltem Wasser in einen Sumpf eines Pflegemittelbehälters des Reinigungsgeräts anzusteuern. Nach dem Schritt 410 des Bestimmens wird optional der Schritt 425 des Generierens ausgeführt.

Das Verfahren 400 kann unter Verwendung eines Ausführungsbeispiels des vorstehend genannten Steuergeräts angesteuert und/oder ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (400) zum Ansteuern eines Erwärmens eines Reinigungsguts (305) während eines Reinigungsvorgangs in einem Reinigungsgerät (300), wobei das Verfahren (400) zumindest folgende Schritte aufweist:
- Einlesen (405) eines Beladungssignals (115), das zumindest einen Beladungsparameter von in dem Reinigungsgerät (300) aufgenommenem Reinigungsgut (305) repräsentiert;
- Bestimmen (410) eines Erwärmungssignals (120) unter Verwendung des Beladungssignals (115), um ein Erwärmen des in dem Reinigungsgerät (300) aufgenommenen Reinigungsguts (305) nach einem letztmaligen Hauptreinigungsvorgang mit einem Pflegemittel anzusteuern;
- **gekennzeichnet durch** den weiteren Schritt (425) des Generierens eines Kaltwassersignals (230), wobei das Kaltwassersignal (230) unter Verwendung des Erwärmungssignals (120) generiert wird, wobei das Kaltwassersignal (230) ausgebildet ist, ein Einleiten von kaltem Wasser nach dem Erwärmen des Reinigungsguts in einen Sumpf eines Pflegemittelbehälters (315) des Reinigungsgeräts (300) anzusteuern.

2. Verfahren (400) gemäß Anspruch 1, mit einem Schritt (415) des Ermittelns des Beladungssignals (115), wobei als Beladungsparameter eine Beladungsmenge und/oder eine Art des in dem Reinigungsgerät (300) aufgenommenen Reinigungsguts (305) ermittelt werden, insbesondere um unter Verwendung einer vorbestimmten Verarbeitungsvorschrift eine in dem Reinigungsgut (305) befindliche oder aufgenommene Wassermenge zu ermitteln.

3. Verfahren (400) gemäß einem der vorangegangenen Ansprüche, mit einem Schritt (420) des Ausgebens eines Belüftungssignals (220) an eine Schnittstelle (225) zu einer Belüftungseinrichtung (335) des Reinigungsgeräts (300), wobei das Belüftungssignal unter Verwendung des Erwärmungssignals (120) bestimmt wird, wobei das Belüftungssignals (220) ausgebildet ist, die Belüftungseinrichtung (335) anzusteuern.

4. Verfahren (400) gemäß einem der vorangegangenen Ansprüche, wobei im Schritt (405) des Einlesens ein Temperatursignal (240) eingelesen wird, das eine erfasste Temperatur des in dem Reinigungsgerät (300) aufgenommenen Reinigungsguts (305) repräsentiert, wobei im Schritt (410) des Bestimmens das Erwärmungssignal (120) unter Verwendung des Temperatursignals (240) bestimmt wird.

5. Verfahren (400) gemäß Anspruch 4, wobei im Schritt (410) des Bestimmens unter Verwendung des Beladungssignals (115), des Temperatursignals (240) und einer Zuordnungsvorschrift eine Heizenergiemenge zum Bestimmen (410) des Erwärmungssignals (120) bestimmt wird, wobei die Zuordnungsvorschrift eine Heizenergiemenge unter Berücksichtigung einer Temperatur des Reinigungsguts (305) und einer Restwassermenge in dem Reinigungsgut (305) abbildet.

## Claims

1. Method (400) for controlling heating of an item (305) to be cleaned during a cleaning process in a cleaning appliance (300), the method (400) comprising at least the following steps:
- reading in (405) a loading signal (115), which represents at least one loading parameter of items (305) to be cleaned received in the cleaning appliance (300);
- determining (410) a heating signal (120) using the loading signal (115) in order to control heating of the item (305) to be cleaned received in the cleaning appliance (300) after a last main cleaning process using a care product;
- **characterised by** the further step (425) of generating a cold water signal (230), the cold water signal (230) being generated using the heating signal (120), the cold water signal (230) being designed to control the introduction of cold water, after heating the item to be cleaned, into a sump of a care product container (315) of the cleaning appliance (300).

2. Method (400) according to claim 1, comprising a step (415) of ascertaining the loading signal (115), wherein a load amount and/or a type of the item (305) to be cleaned received in the cleaning appliance (300) are ascertained as the loading parameter, in particular by using a predetermined processing specification to ascertain an amount of water in or absorbed by the item (305) to be cleaned.

3. Method (400) according to any of the preceding claims, comprising a step (420) of outputting a ventilation signal (220) to an interface (225) for a ventilation device (335) of the cleaning appliance (300), wherein the ventilation signal is determined using the heating signal (120), wherein the ventilation signal (220) is designed to control the ventilation device (335).

4. Method (400) according to any of the preceding claims, wherein, in the step (405) of reading in, a temperature signal (240) is read in, which represents a detected temperature of the item (305) to be cleaned received in the cleaning appliance (300), wherein, in the determining step (410), the heating signal (120) is determined using the temperature signal (240).

5. Method (400) according to claim 4, wherein, in the determining step (410), an amount of heating energy for determining (410) the heating signal (120) is determined using the loading signal (115), the temperature signal (240) and an assignment specification, wherein the assignment specification represents an amount of heating energy taking into account a temperature of the item (305) to be cleaned and a residual amount of water in the item (305) to be cleaned.

## Revendications

1. Procédé (400) permettant de commander le chauffage d'un article à nettoyer (305) pendant un processus de nettoyage dans un appareil de nettoyage (300), le procédé (400) comprenant au moins les étapes suivantes :
- lecture (405) d'un signal de chargement (115) qui représente au moins un paramètre de chargement d'un article à nettoyer (305) reçu dans l'appareil de nettoyage (300) ;
- détermination (410) d'un signal de chauffage (120) à l'aide du signal de chargement (115) pour commander le chauffage de l'article à nettoyer (305) reçu dans l'appareil de nettoyage (300) après un dernier processus de nettoyage principal avec un produit d'entretien ;
- **caractérisé par** l'étape supplémentaire (425) de génération d'un signal d'eau froide (230), le signal d'eau froide (230) étant généré à l'aide du signal de chauffage (120), le signal d'eau froide (230) étant configuré pour commander l'introduction d'eau froide dans un puisard d'un récipient de produit d'entretien (315) de l'appareil de nettoyage (300) après le chauffage de l'article à nettoyer.

2. Procédé (400) selon la revendication 1, comportant une étape (415) de détermination du signal de chargement (115), une quantité de chargement et/ou un type de l'article à nettoyer (305) reçu dans l'appareil de nettoyage (300) étant définis comme paramètre de chargement, en particulier pour définir une quantité d'eau se trouvant ou reçue par l'article à nettoyer (305) à l'aide d'une spécification de traitement prédéfinie.

3. Procédé (400) selon l'une des revendications précédentes, comportant une étape (420) d'envoi d'un signal de ventilation (220) à une interface (225) vers un dispositif de ventilation (335) de l'appareil de nettoyage (300), le signal de ventilation étant déterminé à l'aide du signal de chauffage (120), le signal de ventilation (220) étant configuré pour commander le dispositif de ventilation (335).

4. Procédé (400) selon l'une des revendications précédentes, dans lequel, à l'étape (405) de lecture, un signal de température (240) est lu, lequel représente une température détectée de l'article à nettoyer (305) reçu dans l'appareil de nettoyage (300), à l'étape (410) de détermination, le signal de chauffage (120) étant déterminé à l'aide du signal de température (240).

5. Procédé (400) selon la revendication 4, dans lequel, à l'étape (410) de détermination, une quantité d'énergie de chauffage permettant de déterminer (410) le signal de chauffage (120) est déterminée à l'aide du signal de chargement (115), du signal de température (240) et d'une spécification d'affectation, la spécification d'affectation reflétant une quantité d'énergie de chauffage en tenant compte d'une température de l'article à nettoyer (305) et d'une quantité d'eau résiduelle dans l'article à nettoyer (305).
